# FASCICULE DE BREVET EUROPEEN

(11) **EP 0 377 260 B1**
(45) Date de publication et mention de la délivrance du brevet: **12.05.1993**
(21) Numéro de dépôt: 89203328.3
(22) Date de dépôt: 28.12.1989
(51) Int. Cl.: C07C 69/675, C07C 67/03, C12P 7/62

(54) **Procédé pour la préparation d'esters de l'acide bêta-hydroxybutyrique**
Verfahren zur Herstellung von Estern der beta-Hydroxybuttersäure
Method for the preparation of esters of beta-hydroxybutyric acid

(30) Priorité: 06.01.1989 FR 8900243
(43) Date de publication de la demande: 11.07.1990
(73) Titulaire: SOLVAY, B-1050 Bruxelles (BE)
(72) Inventeur: Pennetreau, Pascal, B-1310 la Hulpe (BE)
(74) Mandataire: Lechien, Monique (FR)

(56) Documents cités:
- EP-A- 0 046 017
- DE-A- 2 822 472
- FR-A- 2 369 242
- US-A- 2 526 554
- HELVETICA CHIMICA ACTA, tome 65, no. 49, 1982, pages 495-503, Publ. Schweizerische Chemische Gesellschaft, Basel, CH; D. SEEBACH et al: "Über die Depolymerisierung von Poly-(R)-3-hydroxy-buttersäureester (PHB)"

## Description

La présente invention concerne un procédé pour la préparation d'esters de l'acide β-hydroxybutyrique par alcoolyse du poly-β-hydroxybutyrate.

On a déjà proposé dans le document Helvetica Chim. Acta 1982, 65 (2), p. 495-503 un procédé pour la préparation d'esters de l'acide β-hydroxybutyrique. Ce procédé comprend une étape d'alcoolyse de polyhydroxybutyrate, isolé ou non de la biomasse, à l'aide de méthanol et d'acide sulfurique en présence d'un solvant tel que le 1,2-dichloréthane, une étape de neutralisation par lavages avec une solution aqueuse de NaCl et une solution aqueuse de NaHCO₃, puis une étape de purification comprenant un séchage sur sulfate de magnésium, une filtration sous pression réduite et une distillation. Les étapes de lavages d'un tel procédé conduisent à des milieux qui ne sont pas aisés à décanter, en particulier du fait de la présence de deux phases, une phase aqueuse et une phase organique, ce qui engendre la formation d'émulsions. Par ailleurs, les temps de réaction, de séparation et de décantation sont très longs.

La présente invention vise à fournir un procédé qui ne présente plus les inconvénients de ce procédé antérieur.

La présente invention concerne à cet effet un procédé pour la préparation d'esters de l'acide β-hydroxybutyrique à partir de poly-β-hydroxybutyrate comprenant :
a) une première étape dans laquelle on effectue une alcoolyse du poly-β-hydroxybutyrate dans un milieu comprenant une phase liquide homogène d'un alcool, d'un solvant halogéné et d'un acide,
b) une deuxième étape dans laquelle on neutralise l'acide avec une base soluble dans la phase liquide homogène, la base utilisée étant un alcoolate,
c) une troisième étape dans laquelle on ajoute un solvant halogéné dans le milieu et on élimine l'eau formée et l'alcool par azéotropie,
d) une quatrième étape dans laquelle on récupère l'ester formé.

Le poly-β-hydroxybutyrate que l'on peut traiter selon l'invention, peut être obtenu de diverses manières. De nombreux microorganismes, notamment des bactéries, sont capables de le synthétiser. La sélection des microorganismes se fait en général sur la base de la quantité relative de poly-β-hydroxybutyrate contenue dans le microorganisme ainsi qu'en fonction de la vitesse de croissance du microorganisme et de sa vitesse de synthèse du poly-β-hydroxybutyrate.

Ces microorganismes peuvent être traités directement, après séparation et séchage subséquent du milieu de culture, pour effectuer l'alcoolyse du poly-β-hydroxybutyrate sans extraction préalable du poly-β-hydroxybutyrate. Cette séparation peut être effectuée par tous les moyens connus à cet effet. Une façon de procéder consiste à centrifuger le milieu de culture de façon à séparer les microorganismes du milieu, à laver avec de l'eau les microorganismes et à sécher subséquemment les microorganismes.

Une autre technique, décrite dans le brevet européen EP 0168095, consiste à extraire le poly-β-hydroxybutyrate à partir d'une suspension aqueuse de microorganismes au moyen d'un solvant qui forme un azéotrope à minimum avec l'eau, l'eau étant éliminée par voie azéotropique.

Une autre technique consiste à traiter les microorganismes avec une solution d'hypochlorite de sodium afin d'obtenir un poly-β-hydroxybutyrate ne contenant plus de débris cellulaires, puis à sécher le produit obtenu.

L'alcool mis en oeuvre dans la première étape est choisi selon le type d'ester que l'on souhaite préparer. N'importe quel type d'alcool peut être utilisé dans le procédé selon l'invention. Généralement, on opère avec des alcools aliphatiques contenant de 1 à 4 atomes de carbone et, plus particulièrement, avec le méthanol, l'éthanol et le propanol. Enfin, de bons résultats ont été obtenus notamment avec le méthanol et l'éthanol.

Lorsqu'on utilise le méthanol dans le procédé, on met habituellement en oeuvre de 0,1 à 50 ml d'alcool par g de poly-β-hydroxybutyrate. De préférence, on met en oeuvre de 0,5 à 20 ml d'alcool par g de poly-β-hydroxybutyrate.

Les solvants halogénés du poly-β-hydroxybutyrate mis en oeuvre dans la première étape sont choisis parmi les solvants du poly-β-hydroxybutyrate miscibles à l'alcool et séparables de l'ester par distillation. On utilise habituellement les chlorométhanes, les chloréthanes et les chloropropanes. Généralement, on utilise le chloroforme, le 1,2-dichloréthane, le 1,2-dichloropropane, le 1,1,2-trichloréthane, le 1,1,2,2-tétrachloréthane et le 1,2,3-trichloropropane. De préférence, on utilise toutefois le 1,2-dichloropropane ou le 1,2-dichloréthane. Enfin, le solvant tout particulièrement préféré est le 1,2-dichloropropane.

Lorsque l'on utilise le 1,2-dichloropropane comme solvant halogéné dans le procédé de l'invention, on met en oeuvre de 0,1 à 50 ml de 1,2-dichloropropane par g de poly-β-hydroxybutyrate et de préférence de 1 à 20 ml de 1,2-dichloropropane par g de poly-β-hydroxybutyrate.

Les acides mis en oeuvre dans la première étape sont ceux couramment utilisés dans l'art antérieur comme catalyseur d'estérification. On utilise habituellement l'acide sulfurique, l'acide p-toluène sulfonique, l'acide méthane sulfonique. Parmi ceux-ci, de bons résultats ont été obtenus avec l'acide sulfurique.

Lorsque l'on met en oeuvre l'acide sulfurique, on utilise habituellement des quantités comprises entre 1 et 5 % en volume d'acide concentré dans l'alcool, de préférence de 2 à 4 %.

Dans le procédé selon l'invention, on travaille dans un mélange réactionnel où le solvant, l'alcool, l'acide et le poly-β-hydroxybutyrate ne constituent qu'une phase liquide homogène, le mélange réactionnel peut toutefois contenir des débris cellulaires et du poly-β-hydroxybutyrate non dissous éventuellement, mais quoi qu'il en soit, on opère en absence d'une seconde phase liquide.

La température à laquelle on effectue la première étape du procédé, est comprise entre 25 et 160°C. Elle est de préférence comprise entre 35 et 150°C; de bons résultats ont été obtenus entre 50 et 140°C.

La pression à laquelle on effectue la première étape du procédé, est généralement comprise entre 1 et 10 bars; elle est habituellement comprise entre 3 et 8 bars. De préférence, on opère sous pression autogène, ce qui implique que la pression de travail est fixée par la composition du milieu réactionnel et par la température choisie.

La première étape du procédé selon l'invention peut être réalisée dans tout appareillage conçu à cet effet.

Lors de la deuxième étape du procédé selon l'invention, on neutralise l'acide avec une base qui est soluble dans la phase liquide homogène et qui forme avec l'acide un sel précipitant au moins partiellement dans le milieu. On met en oeuvre un alcoolate, Habituellement celui d'un métal alcalin ou alcalino-terreux. De préférence, on met en oeuvre l'alcoolate correspondant à l'alcool utilisé dans la première étape et comme métal alcalin ou alcalino-terreux le sodium ou le potassium.

L'addition de la base peut être faite en discontinu. On contrôle la neutralisation en suivant l'évolution du milieu en fonction de la coloration d'un papier pH; l'addition de base est arrêtée lorsque la coloration d'un papier pH correspond à une acidité comprise entre pH 5 et 6 en phase aqueuse.

De préférence, on met en oeuvre la base en solution dans le même alcool qu'utilisé lors de la première étape.

La température à laquelle on effectue la deuxième étape du procédé, est comprise habituellement entre 20 et 50°C et de bons résultats ont été obtenus à température ambiante.

La pression à laquelle on effectue la deuxième étape du procédé, est comprise entre 1 et 3 bars, de bons résultats ont été obtenus à la pression atmosphérique.

La deuxième étape du procédé selon l'invention peut être réalisée dans tout appareillage conçu à cet effet. Habituellement, elle est réalisée dans l'appareillage de la première étape.

Lors de la troisième étape, on ajoute un solvant halogéné dans le milieu en vue d'éliminer l'eau formée et surtout l'alcool par azéotropie.

Comme solvant halogéné, les solvants habituels du poly-β-hydroxybutyrate peuvent être mis en oeuvre. Généralement, on utilise les chlorométhanes, les chloréthanes ou les chloropropanes tels que le chloroforme, le 1,2-dichloréthane, le 1,2-dichloropropane, le 1,1,2-trichloréthane, le 1,1,2,2-tétrachloréthane et le 1,2,3-trichloropropane. De préférence, on met en oeuvre un solvant halogéné identique à celui mis en oeuvre dans la première étape. Dès lors, on utilise de façon particulièrement préférée le 1,2-dichloropropane lors de cette étape.

Pour réaliser l'élimination azéotropique, on ajoute au milieu un volume de solvant halogéné égal au volume d'azéotrope attendu, cette addition peut être réalisée en continu avant ou pendant l'opération de distillation.

L'azéotrope est éliminé à pression atmosphérique ou sous pression réduite. L'élimination du solvant est arrêtée lorsque la température en tête atteint la température d'ébullition du solvant pur.

Lors de la quatrième étape, on récupère l'ester sans autre difficulté par toute technique connue, telle que notamment une filtration et/ou une centrifugation, suivie d'un lavage réalisé de préférence avec un solvant halogéné identique à celui utilisé lors de la première ou de la troisième étape, suivie d'une distillation du solvant halogéné et d'une distillation de l'ester de l'acide β-hydroxybutyrique.

L'ester de l'acide β-hydroxybutyrique obtenu selon le procédé de l'invention peut être utilisé dans toutes les applications connues de ce produit, c'est-à-dire notamment comme médicament, comme produit intermédiaire dans l'industrie chimique fine ou comme additif dans la nourriture animale.

L'ester de l'acide β-hydroxybutyrique obtenu selon le procédé de l'invention peut être utilisé tel quel en synthèse chimique ou être hydrolysé en vue d'obtenir l'acide β-hydroxybutyrique avec une formation minimale d'acide crotonique.

L'exemple qui suit sert à illustrer l'invention.

### Exemple 1

Dans un autoclave de 1 litre, équipé d'un manomètre, d'un système de chauffage et de contrôle de la température, d'une agitation et d'un tube permettant d'effectuer des prélèvements, on introduit en agi tant 200 ml de 1,2-dichloropropane, 200 ml de méthanol, 6 ml d'acide sulfurique concentré (0,110 mole de H₂SO₄) et 72 g d'une culture de microorganismes qui a été centrifugée, lavée puis séchée et qui contient 60 % de poly-β-hydroxybutyrate, ce qui correspond à 0,5 mole d'unités C₄.

Le réacteur ést purgé à l'azote puis le milieu est chauffé à 110°C pendant 5 heures, la pression passe de 4,75 à 5 bars.

Le chauffage est arrêté et on laisse revenir le milieu réactionnel à température ambiante.

On ajoute alors au milieu 45 ml d'une solution méthanolique 1,3 M en méthanolate de sodium; cette addition neutralise l'acide sulfurique.

Ensuite, on ajoute au milieu 355 ml de 1,2-dichloropropane et on procède à l'élimination azéotropique à pression atmosphérique, le point d'ébullition de l'azéotrope est de 63°C.

L'élimination du 1,2-dichloropropane est arrêtée lorsque la température atteint 96°C, point d'ébullition du 1,2-dichloropropane.

Le milieu est ensuite filtré sur une plaque frittée de porosité P3 en 2 minutes et le résidu est lavé 2 fois par 200 ml de 1,2-dichloropropane.

Le gâteau de filtration est alors séché sous pression réduite [< 1,33.10³ Pa (< 10 mm Hg)] à poids constant. Le gâteau de filtration a un poids humide d'environ 110 g et un poids final d'environ 45 g.

Le 1,2-dichloropropane est éliminé sous pression réduite de 20.10³ Pa (150 mm Hg) puis l'ester de l'acide β-hydroxybutyrique est distillé sous une pression de 2,66.10³ Pa (20 mm Hg). Le point d'ébullition de l'ester méthylique est de 76°C sous 2,66.10³ Pa (20 mm Hg).

On obtient 50 g d'ester méthylique de l'acide β-hydroxybutyrique quasiment pur.

L'ester contient très peu de crotonate de méthyle, de l'ordre de 0,05 à 0,1 g/kg.

Les rendements de distillation s'élèvent à 90 %.

Le 1,2-dichloropropane, récupéré lors des opérations de distillation et de lavage, est recyclé dans le procédé.

## Revendications

1. Procédé pour la préparation d'esters de l'acide β-hydroxybutyrique à partir de poly-β-hydroxybutyrate, ce procédé comprenant :
a) une première étape dans laquelle on effectue une alcoolyse du poly-β-hydroxybutyrate dans un milieu comprenant une phase liquide homogène d'un alcool, d'un solvant halogéné et d'un acide,
b) une deuxième étape dans laquelle on neutralise l'acide avec une base soluble dans la phase liquide homogène,
c) une troisième étape, dans laquelle on élimine l'eau,
d) une quatrième étape dans laquelle on récupère l'ester,
caractérisé en ce que la base utilisée dans la deuxième étape est un alcoolate et que lors de la troisième étape, on ajoute un solvant halogéné dans le milieu et on élimine l'eau formée et l'alcool par azéotropie.

2. Procédé selon la revendication 1, caractérisé en ce que l'alcoolate, utilisé dans la deuxième étape, correspond à l'alcool utilisé dans la première étape.

3. Procédé selon la revendication 1 ou 2, caractérisé en ce que l'alcoolate est mis en oeuvre en solution dans l'alcool utilisé dans la première étape.

4. Procédé selon l'une quelconque des revendications précédentes, caractérisé en ce que la quatrième étape comprend une filtration et/ou une centrifugation.

5. Procédé selon la revendication 4, caractérisé en ce que la quatrième étape comprend une distillation de l'ester.

6. Procédé selon l'une quelconque des revendications précédentes, caractérisé en ce que le solvant halogéné utilisé dans la première étape et dans la troisième étape est choisi, indépendamment l'un de l'autre, parmi les chlorométhanes, les chloréthanes et les chloropropanes.

7. Procédé selon la revendication 6, caractérisé en ce que le solvant halogéné utilisé dans la première étape et le solvant halogéné utilisé dans la troisième étape sont identiques.

8. Procédé selon la revendication 7, caractérisé en ce que le solvant halogéné est le 1,2-dichloropropane.

## Claims

1. Process for the preparation of β-hydroxybutyric acid esters from poly-β-hydroxybutyrate, this process comprising:
a) a first step in which an alcoholysis of the poly-β-hydroxybutyrate is carried out in a medium comprising a homogeneous liquid phase of an alcohol, a halogenated solvent and an acid,
b) a second step in which the acid is neutralized with a base soluble in the homogeneous liquid phase,
c) a third step, in which the water is removed, and
d) a fourth step in which the ester is recovered,
characterized in that the base used in the second step is an alcoholate and in that, during the third step, a halogenated solvent is added to the mixture and the water formed and the alcohol are removed by azeotropic distillation.

2. Process according to Claim 1, characterized in that the alcoholate, used in the second step, corresponds to the alcohol used in the first step.

3. Process according to Claim 1 or 2, characterized in that the alcoholate is used in solution in the alcohol used in the first step.

4. Process according to any one of the preceding claims, characterized in that the fourth step comprises filtration and/or centrifugation.

5. Process according to Claim 4, characterized in that the fourth step comprises a distillation of the ester.

6. Process according to any one of the preceding claims, characterized in that the halogenated solvent used in the first step and in the third step is chosen, independently of one another, from the chloromethanes, the chloroethanes and the chloropropanes.

7. Process according to Claim 6, characterized in that the halogenated solvent used in the first step and the halogenated solvent used in the third step are identical.

8. Process according to Claim 7, characterized in that the halogenated solvent is 1,2-dichloropropane.

## Patentansprüche

1. Verfahren zur Herstellung von Estern der β-Hydroxybuttersäure ausgehend von Poly-β-hydroxybutyrat, wobei das Verfahren folgende Stufen umfaßt:
a) eine erste Stufe, in der man eine Alkoholyse des Poly-β-hydroxybutyrats durchführt in einem Medium, das eine flüssige, homogene Phase eines Alkohols, eines halogenierten Lösungsmittels und einer Säure umfaßt,
b) eine zweite Stufe, in der man die Säure mit einer Base neutralisiert, die in der flüssigen, homogenen Phase löslich ist,
c) eine dritte Stufe, in der man das Wasser eliminiert,
d) eine vierte Stufe, in der man den Ester zurückgewinnt,
dadurch gekennzeichnet, daß die in der zweiten Stufe verwendete Base ein Alkoholat ist, und daß man bei der dritten Stufe ein halogeniertes Lösungsmittel dem Medium zufügt und das gebildete Wasser und den Alkohol durch Azeotropie eliminiert.

2. Verfahren nach Anspruch 1, dadurch gekennzeichnet, daß das in der zweiten Stufe verwendete Alkoholat dem in der ersten Stufe verwendeten Alkohol entspricht.

3. Verfahren nach Anspruch 1 oder 2, dadurch gekennzeichnet, daß das Alkoholat in Lösung in dem in der ersten Stufe verwendeten Alkohol eingesetzt wird.

4. Verfahren nach einem der vorhergehenden Ansprüche, dadurch gekennzeichnet, daß die vierte Stufe eine Filtration und/oder eine Zentrifugation umfaßt.

5. Verfahren nach Anspruch 4, dadurch gekennzeichnet, daß die vierte Stufe eine Destillation des Esters umfaßt.

6. Verfahren nach einem der vorhergehenden Ansprüche, dadurch gekennzeichnet, daß das in der ersten Stufe und in der dritten Stufe verwendete halogenierte Lösungsmittel ausgewählt ist, unabhängig voneinander, unter den Chlormethanen, den Chlorethanen und den Chlorpropanen.

7. Verfahren nach Anspruch 6, dadurch gekennzeichnet, daß das in der ersten Stufe verwendete halogenierte Lösungsmittel und das in der dritten Stufe verwendete halogenierte Lösungsmittel identisch sind.

8. Verfahren nach Anspruch 7, dadurch gekennzeichnet, daß das halogenierte Lösungsmittel das 1,2-Dichlorpropan ist.
